# EUROPEAN PATENT SPECIFICATION

(11) **EP 1 757 615 B1**
(45) Date of publication and mention of the grant of the patent: **24.08.2011**
(21) Application number: 05255194.2
(22) Date of filing: 24.08.2005
(51) Int. Cl.: C07K 14/025, C07K 14/21, A61K 39/12, C12N 15/62, C07K 16/00

(54) **Fusion protein for inhibiting cervical cancer**
Fusionsprotein zur Inhibierung von Gebärmutterhalskrebs
Protéine de fusion pour inhiber le cancer du cervix

(43) Date of publication of application: 28.02.2007
(73) Proprietor: Healthbanks Biotech Co., Ltd., Taipei City (TW)
(72) Inventor: Chang, Hsiu-Kang, Taipei City 106 (TW); Liao, Chao-We, Miaoli County 350 (TW); Cheng, Wen-Fang, Taipei City 110 (TW)
(74) Representative: Tranter, Andrew David

(56) References cited:
- WO-A-03/080669
- WO-A-03/085085
- US-A- 5 705 163
- US-A1- 2004 247 617
- US-B1- 6 214 541
- HUNG C-F ET AL: "Cancer immunotherapy using a DNA vaccine encoding the translocation domain of a bacterial toxin linked to a tumor antigen" CANCER RESEARCH, AMERICAN ASSOCIATION FOR CANCER RESEARCH, BALTIMORE, MD, US, vol. 61, no. 9, 1 May 2001 (2001-05-01), pages 3698-3703, XP002354120 ISSN: 0008-5472
- LIAO CHAO-WEI ET AL: "Fusion protein vaccine by domains of bacterial exotoxin linked with a tumor antigen generates potent immunologic responses and antitumor effects" CANCER RESEARCH, vol. 65, no. 19, October 2005 (2005-10), pages 9089-9098, XP002374092 ISSN: 0008-5472
- QIN Y ET AL: "Human papillomavirus type 16 E7 peptide<38-61> linked with an immunoglobulin G fragment provides protective immunity in mice" GYNECOLOGIC ONCOLOGY, ACADEMIC PRESS, LONDON, GB, vol. 96, no. 2, February 2005 (2005-02), pages 475-483, XP004725798 ISSN: 0090-8258
- VOTH D AND BALLARD J: "Clostridium difficile toxins: Mechanism of action and role in disease", CLINICAL MICROBIOLOGY REVIEWS, vol. 18, no. 2, April 2005 (2005-04), pages 247-263,

### SEQUENCE LISTING

<110> HealthBanks Biotech CO. LTD.
<120> Fusion protein for inhibiting cervcal cancer
<130> ADT0638
<160> 14
<170> PatentIn version 3.3
<210> 1
   <211> 310
   <212> DNA
   <213> Human papillomavirus type 16
<400> 1
   ggaattcatg cacggtgaca ccccgaccct gcacgaatac atgctggacc tccagccgga      60
   aaccaccgac ctgtactgct acgaacagct gaacgactcc tccgaagaag aagacgaaat     120
   cgacggtccg gctggtcagg ctgaaccgga ccgtgctcac tacaacatcg ttaccttctg     180
   ctgcaaatgc gactccaccc tgctgctgtg cgttcagtcc acccacgttg acatccgtac     240
   cctggaagac ctgctgatgg gtaccctggg tatcgtttgc ccgatctgct cccagaaacc     300
   gctcgagaaa                                                            310
<210> 2
   <211> 18
   <212> PRT
   <213> Pseudomonas sp.
<400> 2
   Lys Leu Asp Tyr Leu Lys Lys Asp Glu Leu Arg Asp Glu Leu Lys Asp
   1               5                   10                  15
   Glu Leu
<210> 3
   <211> 98
   <212> PRT
   <213> Human papillomavirus type 16
<400> 3
   Met His Gly Asp Thr Pro Thr Leu His Glu Tyr Met Leu Asp Leu Gln
   1               5                   10                  15
   Pro Glu Thr Thr Asp Leu Tyr Cys Tyr Glu Gln Leu Asn Asp Ser Ser
               20                  25                  30
   Glu Glu Glu Asp Glu Ile Asp Gly Pro Ala Gly Gln Ala Glu Pro Asp
           35                  40                  45
   Arg Ala His Tyr Asn Ile Val Thr Phe Cys Cys Lys Cys Asp Ser Thr
       50                  55                  60
   Leu Leu Leu Cys Val Gln Ser Thr His Val Asp Ile Arg Thr Leu Glu
   65                  70                  75                  80
   Asp Leu Leu Met Gly Thr Leu Gly Ile Val Cys Pro Ile Cys Ser Gln
                   85                  90                  95
   Lys Pro
<210> 4
   <211> 52
   <212> DNA
   <213> Artificial
<220>
   <223> artificial primer for E7 peptide.
<400> 4
   agaattcatg cacggtgaca ccccgaccct gcacgaatac atgctggacc tc 52
<210> 5
   <211> 46
   <212> DNA
   <213> Artificial
<220>
   <223> artificial primer for E7 peptide.
<400> 5
   cgtagcagta caggtcggtg gtttccggct ggaggtccag catgta 46
<210> 6
   <211> 51
   <212> DNA
   <213> Artificial
<220>
   <223> artificial primer for E7 peptide.
<400> 6
   ttcgtcttct tcttcggagg agtcgttcag ctgttcgtag cagtacaggt c 51
<210> 7
   <211> 48
   <212> DNA
   <213> Artificial
<220>
   <223> artificial primer for E7 peptide.
<400> 7
   gtccggttca gcctgaccag ccggaccgtc gatttcgtct tcttcttc 48
<210> 8
   <211> 49
   <212> DNA
   <213> Artificial
<220>
   <223> artificial primer for E7 peptide.
<400> 8
   tgcagcagaa ggtaacgatg ttgtagtgag cagcgtccgg ttcagcctg 49
<210> 9
   <211> 48
   <212> DNA
   <213> Artificial
<220>
   <223> artificial primer for E7 peptide.
<400> 9
   ctgaacgcac agacgcaggg tggagtcgca tttgcagcag aaggtaac 48
<210> 10
   <211> 45
   <212> DNA
   <213> Artificial
<220>
   <223> artificial primer for E7 peptide.
<400> 10
   ttccagggta cggatgtcaa cgtgggtgga ctgaacgcac agacg 45
<210> 11
   <211> 46
   <212> DNA
   <213> Artificial
<220>
   <223> artificial primer for E7 peptide.
<400> 11
   aacgataccc agggtaccca tcagcaggtc ttccagggta cggatv 46
<210> 12
   <211> 50
   <212> DNA
   <213> Artificial
<220>
   <223> artificial primer for E7 peptide.
<400> 12
   tttgaattcc ggtttctggg agcagatcgg gcaaacgata cccagggtac 50
<210> 13
   <211> 46
   <212> DNA
   <213> Artificial
<220>
   <223> artificial primer for KDEL.
<400> 13
   agaattcgtc gactacctca aaaaagacga actgagagat gaactg 46
<210> 14
   <211> 50
   <212> DNA
   <213> Artificial
<220>
   <223> artificial primer for KDEL.
<400> 14
   gtggtggtgc tcgagtcatt acagttcgtc tttcagttca tctctcagtt 50

## Description

### BACKGROUND OF THE INVENTION

### 1. Field of the Invention

The present invention relates to a fusion protein, more particularly, to a fusion protein for inhibiting cervical cancer induced or caused by HPV type 16 and pharmaceutical compositions thereof.

### 2. Description of Related Art

Currently, the incidence ratio of cervical cancer has remained high among all woman cancer patients. There are no any obvious symptoms of general variation of cervical epithelium or early cervical cancer. Although cervical cancer can be successfully treated in its early stages, prevention is still much better than treatment. Therefore, the researchers in this field have been making effort to find out the most efficient way to prevent cervical cancer.

It is already proved that human papillomavirus, HPV is highly related with cervical cancer. Some types (e.g. type 16, or 18) of DNA sequence of HPV had been found in cervical cancer cells, about 75%~100%, but it is still not clear what the mechanism is in causing the cancer. Lately, research has found that the early gene product of virus--protein E6 and E7 of highly dangerous type 16, 18 and 31 of HPV easily combines with the product of genes Rb and p53 and thus reduce the ability of anti-tumor agent. This explains that HPV is not functioning alone when causing cancer but is assisted by environmental factors. Moreover, E7 protein expresses continuously in cervical cancer cells and carcinoma tissues. E7 protein also plays an important role in the process of maintaining shifted malignant tissue phenotype.

The cancer immune therapy is mainly displayed with cell-mediated immunization, assisted by humoral immunization. Cells involved in cell-mediated immunization are cytotoxic T lymphocytes (CTL), NK and macrophages. CTL is triggered by interleukin-2, and then identified by T cells. The major histocompatibility complex (MHC) on the cancer cells with antigen present appears and releases lysozyme to destroy the cancer cells and restrain the proliferation of cancer cells. CTL protection is proved to inhibit cancers caused by HPV. Therefore if it is possible to induce the proliferation of the HPV-antigen-specific CTL, for example, CD 8⁺ T lymphocytes, by enhancing complexes of HPV antigen and MHC class I presenting on cancer cells, the strategy of CTL induction with E7 antigen can be able to control carcinoma cell directly and beneficial for immunological prevention and treatment.

It is proved by research that cervical cancer is able to be prevented by vaccine injection. E7 proteins of HPV are highly common in carcinoma tissues or the tissues before carcinoma damage, therefore E7 protein has the potential for developing as a vaccine. Basically, HPV type 16 and HPV type 18 are serious causes of not only cervical cancer, they are also dangerous factors for inducing lung cancer in females. The carcinogenic proteins E6 and E7 can be transferred to lungs through the circulation of blood and they decompose the anti-tumor proteins produced by gene Rb and p53. Once the anti-tumor genes or the proteins produced by them are deactivated, cancer cells show up. Though the present DNA vaccine does have a long term effect, on the other hand, it has high production costs. The main factor of restrained development in the DNA vaccine is the highly dangerous nature of the virus itself, which mutates easily. Furthermore, when applying E7 protein in gene therapy to cervical cancer, the induced immune response is usually induced weaklybecause of the weak antigen character of E7 protein of HPV virus. The effect of the prophylaxis and the therapy of cervical cancer are not able to be evaluated because of frail immune response.

Generally, the specific antigen of cancer cells needs to bemodified and combined with MHC-I then presented to the cell surface in order to trigger the CD8⁺ cells and elicit cell-mediated system. The research shows that the HPV type 16 E7 gene can be found in cervical cancer tissues but there is a lack of the specific MHC-I complex to present to the cell surface for showing E7 antigen. Therefore, HPV type 16 E7 protein will not be presented to or initiate the cellular immune system of the host cell and then HPV escapes from the detection or monitoring of host. Usually, when E7 protein is injected in vivo, it is considered as external antigens. The E7 vaccination can only induce the humoral immune response thus lowering the effect to elicit cell-mediated immunity. Hence, it is necessary to develop a transportation system of sending the intact foreign protein into cytoplasm and induce effective immune response.

Therefore, it is desirable to provide a fusion protein for inhibiting cervical cancer to mitigate and/or obviate the aforementioned problems.

WO 03/085085 A2 discloses Nucleic acids encoding a chimeric or fusion polypeptide which polypeptide comprises a first domain comprising a translocation polypeptide; and a second domain comprising at least one antigenic peptide are disclosed. The preferred translocation polypeptide is a bacterial toxin translocation polypeptide, such as domain II of *Pseudomonas aeruginosa* exotoxin A (ETA(dII)).

U.S. Patent Publication No. 20040247617 discloses a fusion antigen specific for a target cell comprising a ligand moiety which is capable of reacting, recognizing or binding to the receptors on the target cell, a Pseudomonas exotoxin A translocation domain II, an antigenic moiety, and a carboxyl terminal moiety which permits combination of the fusion antigen to the endoplasmic reticulum (ER) membrane of the target cell.

U.S. Patent No. 5705163 discloses a target-specific, cytotoxic, recombinant Pseudomonas exotoxin. Such toxins are made by inserting specific recognition molecules at specific cloning sites in at least domain III near the carboxyl terminus of the PE molecule.

### SUMMARY OF THE INVENTION

The invention in its broadest sense is as defined in the independent claims. In one aspect, the invention relates to a fusion protein comprising: a) a polypeptide comprising a *Pseudomonas* exotoxin A (PE) fragment, the PE fragment comprising domains I and II but not domain III of the exotoxin; b) a human papillomavirus type 16 (HPV16) E7 protein; and c) a carboxyl terminal section comprising an amino acid sequence selected from the group consisting of KDEL, KDELRDELKDEL and SEQ ID NO: 2, wherein the capital letters used in the terms 'KDEL' and 'KDELRDELKDEL' represent single letter amino acid codes.

In another aspect, the invention relates to a pharmaceutical composition comprising an effective amount of a fusion protein as aforementioned for suppressing cancer induced by HPV 16.

Further in another aspect, the invention relates to a fusion protein as aforementioned for use in inhibiting cancer induced by HPV16.

Yet in another aspect, the invention relates to a pharmaceutical composition as aforementioned for use in inhibiting cancer induced by HPV16.

. The fusion protein of the present invention can effectively inhibit the proliferation of carcinoma cells induced by HPV16 and lower the carcinoma level, and moreover can prevent cancers induced by HPV16.

The fusion protein of the present invention is able to induce CTL and antibody protection *in vivo,* then further is able to destroy the infected cells by presenting the HPV 16 E7 antigen. A pharmaceutical composition for preventing or inhibiting cancer cells induced by human papillomavirus type 16 and comprising an effective amount of the fusion protein of claim 1 is also disclosed in the present invention.

The cancer induced by human papillomavirus type 16 can be inhibited or prevented by the fusion protein of the present invention or the pharmaceutical composition thereof. More precisely, the cancer is cervical cancer or lung cancer. In the fusion protein of the present invention, the nucleotide sequence of E7 peptide segment of human papillomavirus type 16 is preferred as encoded by SEQ ID NO: 1.

The preferable HPV 16 E7 amino acid sequence of fusion protein of the present invention is SEQ ID NO: 3.

The fusion protein of the present invention can be applied for inhibiting or preventing the infection of human papillomavirus type 16. The pharmaceutical composition of the present invention can further include an adjuvant for enhancing the medical effect. The adjuvant can be any conventional adjuvant of the art. Preferably, the adjuvant is aluminum gel, oily adjuvant such as Freund's FCA, or FIA, mannide mono-oleate emulsifier, ISA 206™, or ISA 720™. More preferably, the adjuvant is ISA 206™.

The pharmaceutical composition of the present invention can selectively comprise any conventional adjuvant, dispersant, humectant (for example: Tween 80™) and suspension to produce sterile injection, for example, a sterile injectable composition can be a solution or suspension in a non-toxic parenterally acceptable diluent or solvent, such as a solution in 1,3-butanediol. Among the acceptable vehicles and solvents that can be employed, mannitol or water is preferred. In addition, fixed oils are conventionally employed as a solvent or suspending medium (e.g., synthetic mono- or diglycerides). Fatty acid,(e.g. oleic acid or glyceride derivatives thereof), and pharmaceutically acceptable natural oils (e.g. olive oil or castor oil, especially polyoxyethylated derivatives thereof) can be used in the preparation of injectable compositions. These oil solutions or suspensions can also contain a long chain alcohol diluent or dispersant, carboxymethyl cellulose, or similar dispersing agents. Other commonly used surfactants such as Tweens, Spans, other similar emulsifying agents, or bioavailability enhancers which are commonly used in the manufacture of pharmaceutically acceptable solid, liquid, or other dosage forms can also be used for the purpose of formulation.

A composition for oral administration can be any orally acceptable dosage form including capsules, tablets, emulsions and aqueous suspensions, dispersions, and solutions. In the case of tablets, the preferable vehicle is lactose, or corn starch. Lubricating agents, such as magnesium stearate, are also typically added. For oral administration in a capsule form, the used diluent is preferred to be lactose, or dried corn starch. When aqueous suspensions or emulsions are administered orally, the active ingredient can be suspended or dissolved in an oily phase combined with emulsifying or suspending agents. If necessary, certain sweetening, flavoring, or coloring agents can be added.

The vehicle in the pharmaceutical composition must be "acceptable" in the sense that it is compatible with the active ingredient of the composition (and preferably, capable of stabilizing the active ingredient) and not deleterious to the subject to be treated. Examples of other vehicles include colloidal silicon oxide, magnesium stearate, cellulose, sodium lauryl sulfate, and D&C Yellow # 10.

The fusion protein of the present invention or the pharmaceutical composition thereof can inhibit or prevent the disease induced by the infection of human papillomavirus type 16. Moreover, the concentration of the antibody induced by the fusion protein of the present invention or the pharmaceutical composition in a subject can last for a long time, and further enhance the medical effect.

Other objects, advantages, and novel features of the invention will become more apparent from the following detailed description when taken in conjunction with the accompanying drawings.

### BRIEF DESCRIPTION OF THE DRAWINGS

FIG. 1 shows the flow chart for a 8.0-kb plasmid (named pET-E7-KDEL3) encoding PE(ΔIII)-E7-KDELKDELKDEL (named PE(ΔIII)-E7-KDEL3) construction in example 2;
FIG. 2 shows the result of *in vivo* tumor protection experiments in example 2; 100% of mice receiving PE(ΔIII)-E7-KDEL3 protein remained tumor-free 60 days after the TC-1 challenge. In contrast, all of the unvaccinated mice and mice receiving E7, PE(ΔIII), and PE(ΔIII)-E7 protein groups developed tumors within 15 days after the tumor challenge;
FIG.3 shows the numbers of E7-specific IFN-γ-secreting CD8⁺ T cell precursors in the PE(ΔIII)-E7-KDEL3 group in example 6;
FIG. 4 shows the results for evaluation of the PE(ΔIII)-E7-KDEL3 protein enhancing the titer of anti-E7 antibody in example 7;
FIG. 5 shows the numbers of E7-specific CD 8⁺ T lymphocytes secreting from mice vaccinated with the fusion protein of the present invention with or without an adjuvant in example 8;
FIG. 6 shows the anti-tumor effects in mice with or without an adjuvant in example 8;
FIG. 7A shows the pulmonary tumor nodules in the *in vivo* tumor treatment experiments in example 9, wherein the symbols illustrate: (i) control group, (ii) E7, (iii) PE(ΔIII), (ix) PE(ΔIII)-E7, and (x) PE(ΔIII)-E7-KDEL3;
FIG. 7B shows the anti tumor effects of mice vaccinated with various times ofPE(ΔIII)-E7-KDEL3 protein in example 9;
FIG. 8A shows the tumor prevention effects of mice vaccinated with various times of fusion protein *in vivo* in example 10; and
FIG 8B shows the tumor suppression effects of mice treated with various times of fusion protein *in vivo* in example 10; and

### DETAILED DESCRIPTION OF THE PREFERRED EMBODIMENT

### Example 1: Synthesis of E7 nucleotide and KDEL sequence

HPV type 16 E7 protein sequence (NC_001526, SEQ ID NO: 3 ) was found in the database of National Center for Biotechnology Information (NCBI), U.S.A., 98 amino acid were collected in total.

The method disclosed in Taiwan patent application number TW200512291 was conducted to express HPV 16 E7 protein by *E.coli* system in large scale. Modification of the nucleotides in the present embodiment is to replace single base of wild type virus sequence with another base that expressed well in *E.coli* system, allowing target proteins expressed in *E.coli* the same as that expressed naturally. The modified sequence of HPV16 E7 nucleotide is SEQ ID NO: 1 (see Figure 9).

Eight pairs of primers were used for the synthesis of polynucleotides in the present example. The polynucleotides were synthesized by polymerase chain reaction (PCR). The sequences of all primers are shown in table 1 and Figure 9. The sequence underlined represents a complementary fragment to a specific sequence.

At first, F1 and R1 primers were used to perform polynucleotides synthesis by PCR without DNA template. There are 15 bases designed for complementary to each other at 3' end of the both primers, and a double strand DNA template was obtained thereby. After the first PCR, 1 µl of amplicon was used as DNA template to conduct the second PCR, and 4 µl of primers of F1, R2, required dNTPs, reagent and Pfu polymerase were mixed to perform the second PCR. The modified nucleotide sequence SEQ. ID NO: 1 was synthesized after eight times of PCR as described above.

Signal peptides with KDEL sequence are prepared in the same method illustrated above. The primer sequences are shown as K3F and K3R in table 1. The nucleotide sequence of the synthesized KDEL is SEQ ID NO: 2.

**Table 1**

| Seq. | Primers | SEQ ID NO: | Sequence listing |
|---|---|---|---|
| E7 | F1 | 4 | |
| | R1 | 5 | |
| | R2 | 6 | |
| | R3 | 7 | |
| | R4 | 8 | |
| | R5 | 9 | |
| | R6 | 10 | |
| | R7 | 11 | |
| | R8 | 12 | |
| KDEL | K3F | 13 | |
| | K3R | 14 | |

### Example 2: Construction of plasmids

The E7 product obtained from the PCR in example 1 was separated in 5% polyacrylamide agarose gel. The target product was purified according to the molecular weight of the product. The vector pET or Ppe (ΔIII) was provided (J.R. Chen, C.W. Liao, S, J.T. Mao, and C.N. Weng, Vet. Microbiol. 80 (2001) 347-357) and digested with restriction endonuclease as well as the purified E7. Another electrophoresis was conducted with 5% polyacrylamide agarose gel for further isolation and purification. Then 0.3kb of E7 sequence fragment was obtained. The 7.84 kb plasmid PE (ΔIII) was further constructed by ligating the E7 fragment and the vector, which comprised of exotoxin A (ETA) but without enzyme toxic section. Moreover, the plasmid pPE (ΔIII)-E7 containing the fusion protein PE(ΔIII)-E7, and the 3.83 kb plasmid pE7 containing E7 fragment and pET23a were also constructed.

A 3.78kb pKDEL3 plasmid which encoded KDELRDELKDEL polypeptide fragment was obtained by digestion, purification of the amplicon (obtained from PCR with K3-F, and K3-R primers), and further insertion into the site of Sall-Xhol of the vector pET23a.

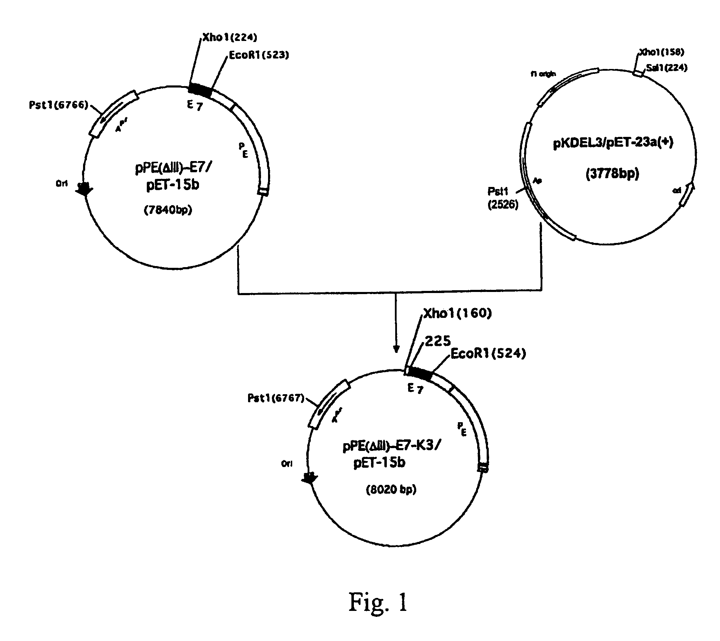

A 8.0 kb plasmid pPE(DIII)-E7-K3 encoding fusion protein PE (ΔIII)-E7-K3 was obtained by digestion of 1.47kb KDEL sequence from pKDEL3 plasmid by restriction endonuclease Sall and Pst1, and further insertion into the spliced 6.5kb, PE (ΔIII)-E7 plasmid DNA which was spliced by Xholl and Pstl. The flow chart of preparing plasmid mentioned above is shown in Figure 1.

The plasmid constructed above was further transfected into *E*. *coli* and maintained in the bacteria strain JM108.

### Example 3: Purification of protein

The plasmid synthesized above was further transfected into *E. coli* BL21 (DE3) pLys strain. The transformed *E. coli* BL21 (DE3) pLys strain was cultured in the 200 ml LB culture medium containing 200 µg/ml ampicillin until the culture concentration reached 0.3 under OD550 spectrum. Then after 1mM IPTG (isopropylthio-ß-D-galactoside, Promege, USA) was added, the *E. coli* BL21 (DE3) pLys strain was cultured for 2 hours. The grown cells were collected by centrifugation. A freeze-thraw method was conducted to the target protein-containing cells to loosen the structure of cell membrane. 10ml of lysis buffer (0.3 mg/ml lysozyme, 1 mM PMSF and 0.06 mg/ml DNAse I) was added to the cultured cells, and then placed at room temperature for 20 minutes. 1ml 10% Triton X-100 was added, and placed at room temperature for 10 minutes. The target proteins were released and collected by centrifugation at a rate of 1200xg for 10 minutes, resulting pallet was washed with 1M or 2M urea. At the end, the collected protein of inclusion body was dissolved in 8 ml of 8M urea.

The fusion proteins were then purified under the His-Tag system in the denatured condition according to the manufacturer's manual (Novagen, USA). The denatured samples in 8M urea were loaded into a column packed with NTA-Ni2⁺ -bind agarose resin. The bound proteins were then eluted with different pH buffer (from 8.0, 7.0, 6.5, 6.0, 5, 4, and 3.5) containing 6M urea, 0.3M NaCl, and 20mM Tris-HCL and 20mM phosphate buffer. After purification, protein elution fractions were analyzed for the purity and quantitated by SDS-PAGE analysis as described previously. The purified protein product contained the amino acid sequence of SEQ ID NO: 3.

### Example 4: preparing carcinoma cell strain (TC-1)

HPV16 E6, E7 and *ras* oncogene were used to transform primary lung epithelial cells of C57BL/6 mice. This tumorigenic cell line was named TC-1. TC-1 cells were grown in RPMI 1640, supplemented with 10% (vol/vol) fetal bovine serum, 50 units/ml penicillin/ streptomycin, 2mM L-glutamine, 1 mM sodium pyruvate, 2mM nonessential amino acids and 0.4 mg/ml G418 at 37°C with 5% CO₂. On the day of tumor challenge, tumor cells were harvested by trypsinization, washed twice with 1X Hanks buffered salt solution (HBSS) and finally resuspended in 1X HBSS to the designated concentration for injection.

### Example 5 : In vivo tumor protection experiments

The testing protein samples: E7, PE (ΔIII), PE (ΔIII)-E7, PE(ΔIII)-E7-KDEL3 were diluted with a phosphate buffer solution at a ratio of 1:10 to make the concentration at 0.1 mg/ml. Then the test samples were incubated at 37°C for 2 hours. The incubated samples were mixed with 10% ISA206™ (Sepec, France) by a vortex to form 4 kinds of different vaccines. Then 0.1 mg of each vaccine obtained was injected into the mice for vaccination. These mice were then boosted subcutaneously two weeks later with the same regimen as the first vaccination. One week after the last vaccination, mice were challenged with 5x10⁴ TC-1 tumor cells by subcutaneous injection in the right leg. Naive mice received the same amount of TC-1 cells to assess natural tumor growth as a control. Tumor growth was monitored by visual inspection and palpation twice weekly until 7, 14, 20, 30, and 60 days after tumor challenge. The spleens of the sacrificed mice were also taken out for further checking.

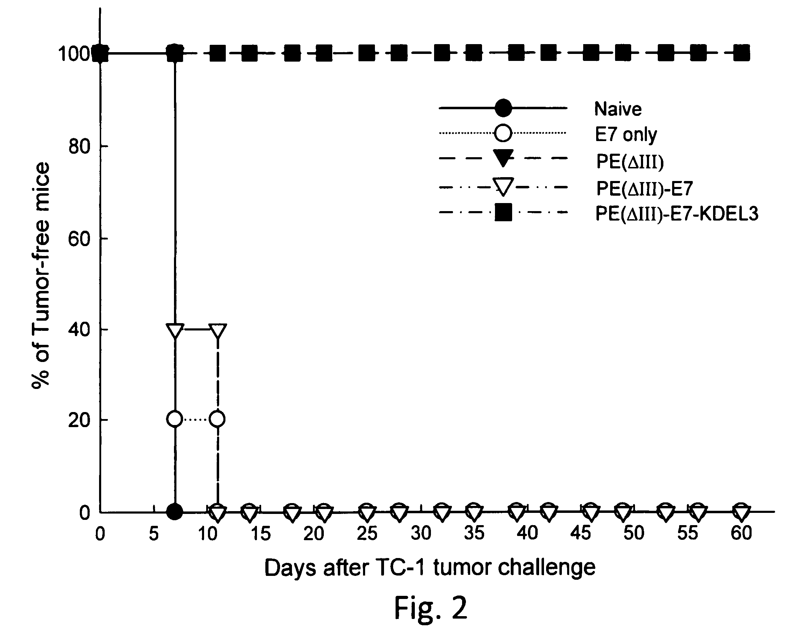

As shown in Fig. 2, no cancer cells were found in the mice injected with PE (ΔIII)-E7-KDEL3. In other words, the percentage of the PE(ΔIII)-E7-KDEL3-injected mice without cancer cells was 100 %. Moreover, even 60 days later, none of the PE(ΔIII)-E7-KDEL3-injected mice had cancer. In contrast, cancer cells could be found in the mice injected with E7, PE(ΔIII), or PE(ΔIII)-E7, or the control mice. The longest period without cancer cells among these mice was 20 days. According to the result of the experiment, only fusion protein that included the sequences of PE (ΔIII), and KDEL3, and the fragment of E7 could prevent and inhibit the growth of cancer cells in the cancer-inducing model illustrated above.

### Example 6: Cell immune experiment

Mice were injected and cancer-induced as described in example 5. One week later, the mice were sacrificed and the spleen macrophages were isolated. Before intracellular cytokine staining, 3.5x10⁵ pooled splenocytes from each vaccinated group were incubated for 16 hours with either 1 µg/ml of E7 peptide (aa 49-57) containing an MHC class I epitope for detecting E7-specific CD8⁺ T cell precursors. Cell surface marker staining of CD8⁺ or CD4⁺ and intracellular cytokine staining for IFN-γ, as well as FACScan analysis, were performed using conditions described by Cheng, et al.(Hum Gene Ther, 13:553-568, 2002) to compare the E7-sepcific immunological assays in mice receiving different regimens of vaccination.

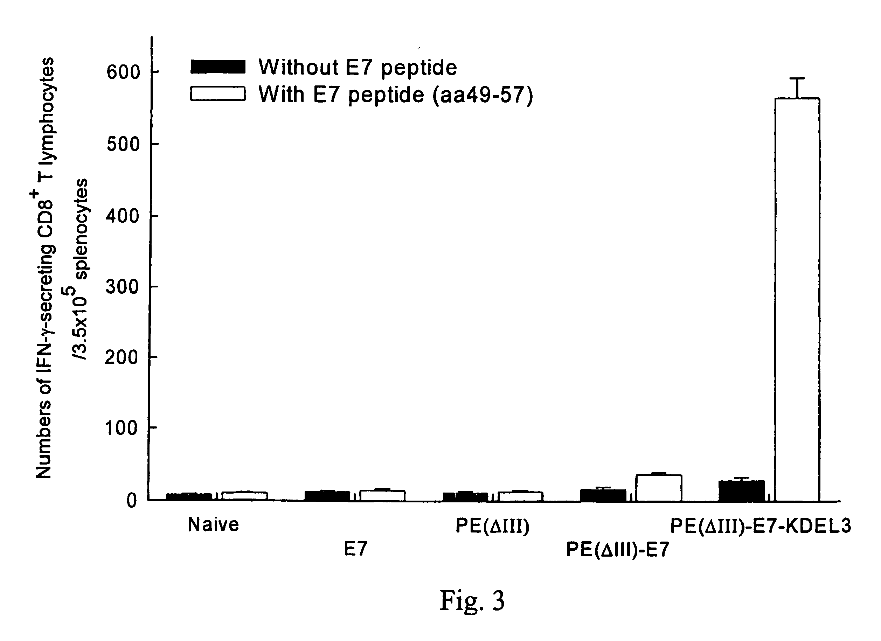

In the present example, it is confirmed that PE(ΔIII)-E7-KDEL3 has influence for E7 specific immunization, as shown in figure 3. In the mice of the group injected with PE(ΔIII)-E7-KDEL3, it was founded that the numbers of E7-specific IFN-γ-secreting CD8⁺ T cell precursors in the PE(ΔIII)-E7-KDEL3 group were higher than those in the other groups (10.0±1.4 in the naïve group, 14.0±2.1 in the E7 group, 12.0±2.1 in the PE(ΔIII) group, 36.0±2.8 in the PE(ΔIII)-E7 group and 564.0±28.0 in the PE(ΔIII)-E7-KDEL3, p<0.01, ANOVA).

According to the result above, the number of E7-specific IFN-γ(+) CD8⁺ T cell precursors of the mice vaccinated with PE(ΔIII)-E7-KDEL3 protein was 40 times higher than that vaccinated with E7.

### Example 7: E7 specific antibody evaluation

Mice were vaccinated with 0.1 mg of the E7, PE(ΔIII), PE(ΔIII)-E7, or PE(ΔIII)-E7-KDEL3 fusion proteins as described in example 5. Further boosts at one and two weeks later with the same regimen as the first vaccination were conducted. The mouse serum was collected at the 7^{th} day after the last immunization.

Briefly, a 96-microwell plate was coated with 100 µl of bacteria-derived HPV-16 E7 proteins (0.5 µg/ml) and incubated at 4°C overnight. The wells were then blocked with phosphate-buffered saline (PBS) containing 20% feta bovine serum. Sera prepared from mice of various vaccinated groups were serially diluted in PBS, added to the ELISA wells, and incubated at 37°C for 2 hr. After washing with PBS containing 0.05% Tween 20™, the plate was incubated with a 1:2000 dilution of a peroxidase-conjugated rabbit anti-mouse IgG antibody (Zymed, San Francisco, CA) at room temperature for 1 hr. The plate was washed, developed with 1-Step Turbo TMB-ELISA (Pierce, Rockford, IL), and stopped with 1 *M*H₂SO₄. The ELISA plate was read with a standard ELISA reader at 450 nm.

C57BL/6 mice were immunized subcutaneously with PE(ΔIII)-E7-KDEL3 mixed 10% ISA206™ adjuvant one to three times. Sera were prepared and the E7-specific antibody titers were detected by the ELISA as described earlier.

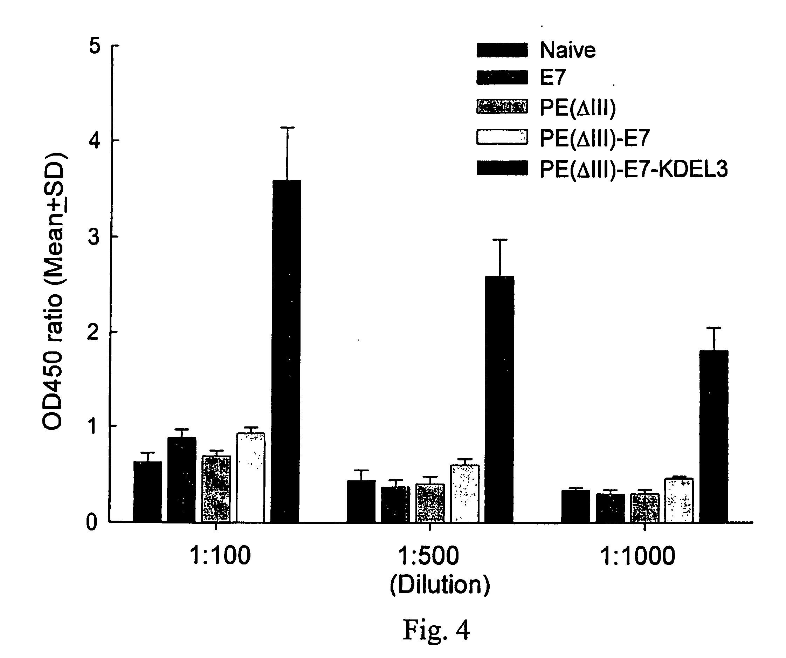

In the present example, it was further confirmed that PE(ΔIII)-E7-KDEL3 was able to improve the potency of resisting E7 antibody. As shown in Figure 4, mice vaccinated with the PE(ΔIII)-KDEL/E7 protein generated the highest titers of anti-E7 Ab's in the sera of mice compared with those vaccinated with other fusion proteins (for 1:100 dilution, 0.629±0.093 in the naïve group, 0.882±0.086 in the E7 group, 0.690±0.06 in the PE(ΔIII) group, 0.930±2.80.06 in the PE(ΔIII)-E7 group and 3.593±0.54 in the PE(ΔIII)-E7-KDEL3 group, p<0.01, AVONA). Apparently, the PE(ΔIII)-E7-KDEL3 protein could also enhance the titer of anti-E7 antibody.

The data showed that PE(ΔIII)-E7-KDEL3 fusion protein could enhance E7-specific immunological responses (including the numbers of E7-specific CD4⁺ and CD8⁺ T lymphocytes and the titers of E7-specific antibodies).

All the obtained readings were expressed with Mean Value and Mean±SEM. The compared data from the experiment were processed using ANOVA analysis by Statistical Package for Social Sciences, SPSS 9.0, SPSS Inc, Chicago, IL; there was a significant difference in the data if the statistical error was under 0.05.

### Example 8 Application of an adjuvant in a vaccine composition

In many cases, peptides or proteins are poorly immunogenic and hardly induce a response when they were injected alone. Hence, an adjuvant is usually injected together with peptides or proteins. Examples of such adjuvants include BCG, incomplete Freund's adjuvant, cwellra toxin B, GM-CSF, ISA206 and IL-12. ISA206™ was used for the protein adjuvant of the present embodiment.

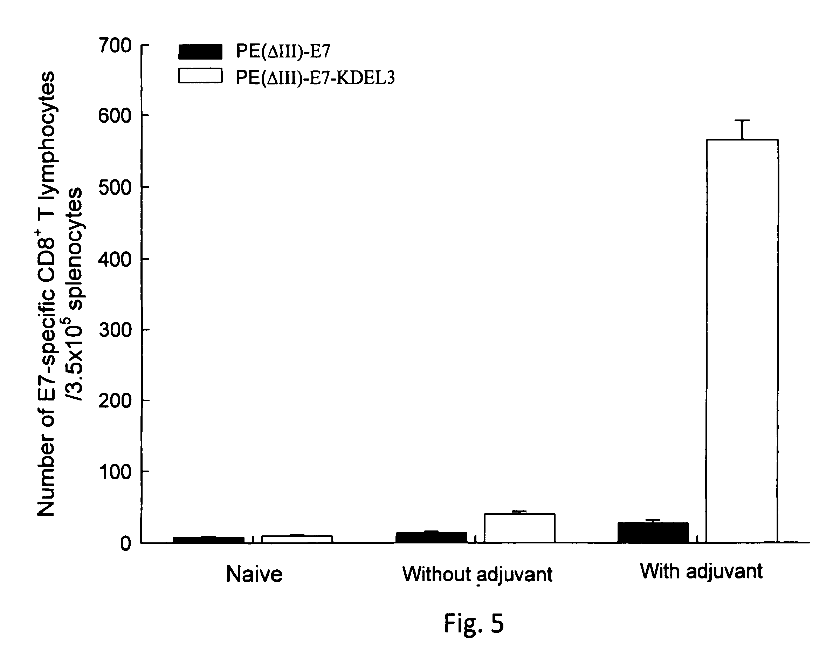

The fusion proteins here were PE(ΔIII)-E7, and PE(ΔIII)-E7-KDEL3. The process of mice vaccination was the same as that described above in examples 5 and 6. Samples of fusion proteins were mixed with or without 10% ISA206™ adjuvant (SEPPIC, France). The result is shown in Fig. 5, wherein the first sample group (i. e., the blank sample group) showed no significant immune response for E7 specific CD 8⁺ T lymphocytes stimulation. The same result could be found in the second sample group. In other words, no matter E7 was included in the vaccine or not, there was no significant numbers of antibody induced by the vaccine composition without adjuvants. However, the numbers of E7 specific CD 8⁺ T lymphocytes was about 600, which was 500-600 times higher than those induced by the vaccine composition without adjuvant.

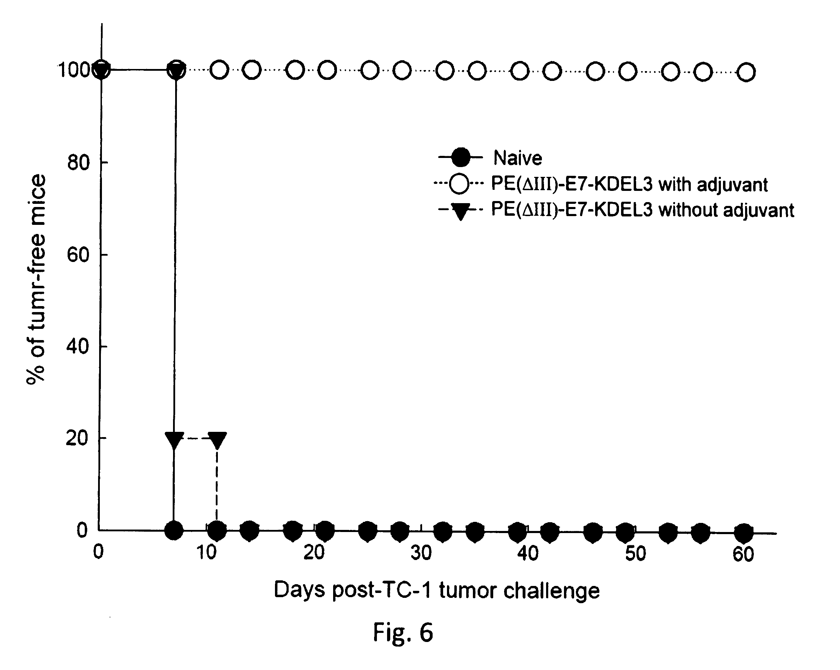

As shown in Fig. 6, the period for preventing the proliferation of cancer in the induced mice by administrating (through injection) the mice with the vaccine composition having PE (ΔIII)-E7-KDEL3 and adjuvant is 60 days. In contrary, for the mice administrated with the vaccine composition of PE(ΔIII)-E7-KDEL3 without an adjuvant, the population of the mice with tumor was almost the same as that of the control group which was not vaccinated with fusion proteins of the present invention. Mice immunized with PE(ΔIII)-E7-KDEL3 protein alone (i.e. without an adjuvant) could not generate potent E7-specific immunological responses and anti-tumor effects (data not shown). However, according to the result, a vaccine composition of PE(ΔIII)-E7-KDEL3 protein of the present invention comprising an adjuvant is preferred for application for capability to induce optimal immunological responses.

### Example 9

*In vivo* tumor treatment experiments were performed using a lung hematogenous spread model. C57BL/6 mice (five per group) were challenged with 5x10⁴ cells/mouse of TC-1 tumor cells via tail vein. Two days after the tumor challenge, mice received 0.1 mg/mouse of E7, PE(ΔIII), PE(ΔIII)-E7 or PE(ΔIII)-E7-KDEL3 protein vaccines subcutaneously, followed by a booster with the same regimen every 7 days for 2 weeks (a total of four times, 0.3 mg protein). Mice receiving no vaccination were used as a negative control. Mice were sacrificed and lungs were explanted on day 30. The pulmonary tumor nodules in each mouse were evaluated and counted by experimenters blinded to sample identity.

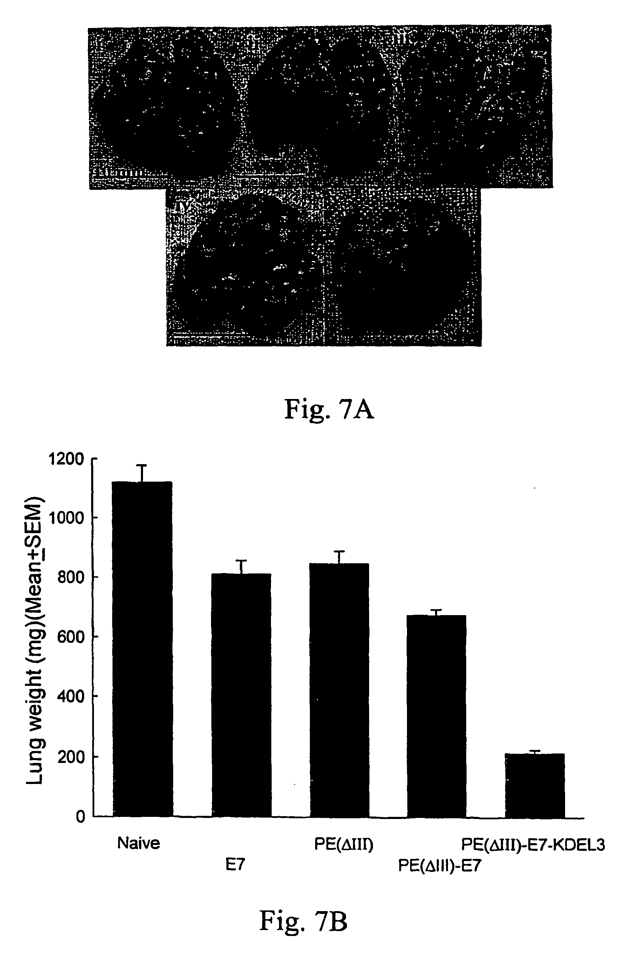

The representative figures of pulmonary tumor nodules in various protein-vaccinated groups are shown in Fig.7A and 7B. As shown in Fig. 7A, only the mice accepting the PE(ΔIII)-E7-KDEL3 fusion protein did not have lung cancer. The mean lung weight (214.4±11.6) of the mice treated with PE(ΔIII)-E7-KDEL3 showed significantly lower than those of mice treated with PE(ΔIII)-E7 (673.6±20.8) or wild-type E7 protein (811.1±45.6) (one-way ANOVA, p<0.001) These data indicated that mice treated with PE(ΔIII)-E7-KDEL3 could control established E7-expressing tumors in the lungs.

### Example 10

Evaluation of the E7-specific immunological profiles of the mice immunized with different times of PE(ΔIII)-E7-KDEL3 protein vaccine could reflect the anti tumor effects of the mice. As described earlier in examples 5 and 6, mice were challenged with TC-1 tumor cells and then received 0.1 mg PE(ΔIII)-E7-KDEL3 protein from one to three times as described earlier. Mice were sacrificed on day 30 and the pulmonary tumor nodules in each mouse were evaluated and counted as described earlier.

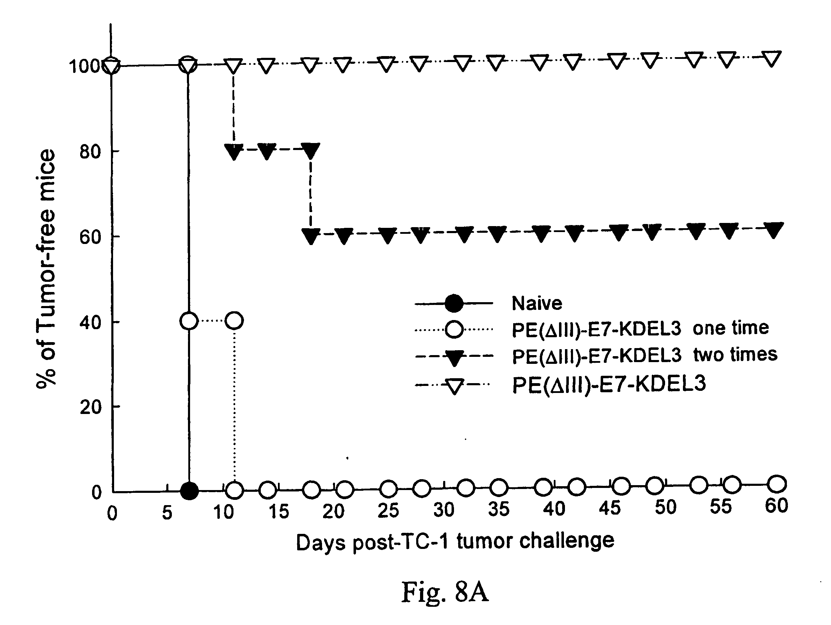

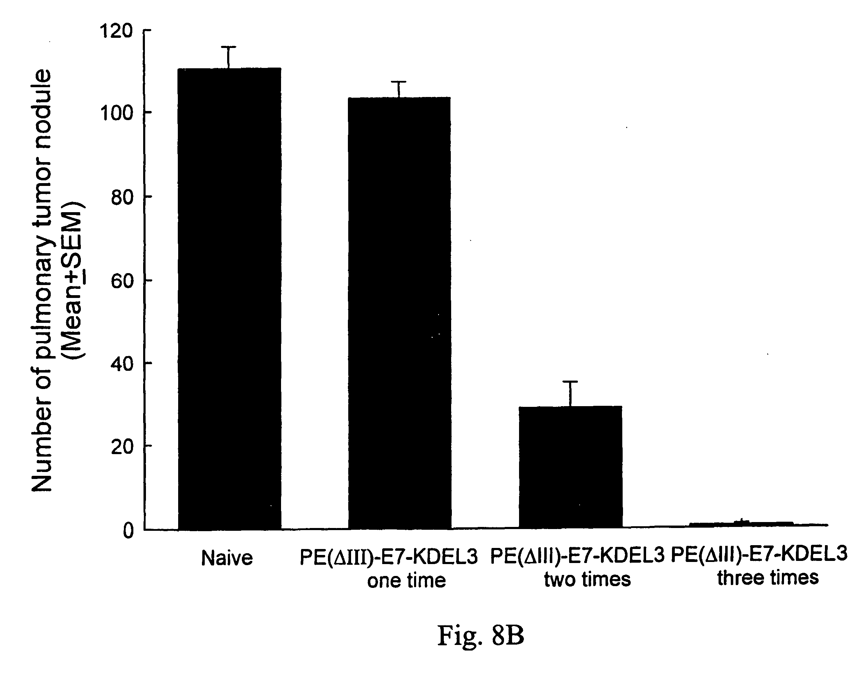

As shown in Fig. 8A, all of the naive mice and mice immunized one time of PE(ΔIII)-KDEL3 protein vaccine grew tumors within 14 days after tumor cell TC-1 challenge. And 60% or 100% of mice immunized with 2 or 3 times of PE(ΔIII)-KDEL3 protein vaccine were tumor-free 60 days after tumor challenge, respectively.

Similar phenomena were also observed in the tumor treatment experiments as described in example 9. The pulmonary tumor nodules decreased significantly from one to three shots of PE(ΔIII)-KDEL3 protein vaccine (103.0±3.8 for one time, 28.8±6.1 for two times, 0.6±0.4 for three times, p<0.001, ANOVA)

Our results show that increasing shots of PE(ΔIII)-KDEL3 protein vaccine could improve the preventive and therapeutic anti-tumor effects on E7-expressing tumor cells.

PE(ΔIII)-E7-KDEL protein could enhance MHC class I presentation of E7 in cells expressing this fusion protein to enhance E7-specific CD8⁺ T-cell activity in vivo.

According to the examples illustrated above, the fusion protein of the present invention can enhance the stimulation of the precursor of E7 specific CD 8⁺ T lymphocytes and CD 4⁺ T lymphocytes by enhancing the presentation of the E7 antigen through MHC I and II. The concentration of the E7 specific antibody can be increased through the mechanism illustrated above. Moreover, the cancer induced by E7 can be inhibited or prevented through the administration of the fusion protein of the present invention. In addition, the mice vaccinated by the fusion protein of the present invention have longer time for inhibiting cancer.

## Claims

1. A fusion protein comprising:
a) a polypeptide comprising a *Pseudomonas* exotoxin A (PE) fragment, the PE fragment comprising domains I and II but not domain III of the exotoxin;
b) a human papillomavirus type 16 (HPV16) E7 protein; and
c) a carboxyl terminal section comprising an amino acid sequence selected from the group consisting of KDEL, KDELRDELKDEL and SEQ ID NO: 2, wherein the capital letters used in the terms 'KDEL' and 'KDELRDELKDEL' represent single letter amino acid codes.

2. The fusion protein of claim 1, wherein the carboxyl terminal section comprises the amino acid sequence of KDELRDELKDEL.

3. A fusion protein according to claim 1, or 2, wherein the HPV16 E7 protein comprises the amino acid sequence of SEQ ID NO: 3.

4. A fusion protein according to claim 1, wherein said carboxyl terminal section comprises the amino acid sequence of KDEL.

5. A fusion protein according to claim 1, wherein the carboxyl terminal section comprises the amino acid sequence of SEQ ID NO: 2.

6. A pharmaceutical composition comprising an effective amount of a fusion protein according to any of claims 1-5 for suppressing cancer induced by HPV16.

7. A pharmaceutical composition according to claim 6, further comprising an adjuvant.

8. A pharmaceutical composition according to claim 7, wherein the adjuvant is an oil adjuvant.

9. A fusion protein according to any of claims 1-5 for use in inhibiting cancer induced by HPV16.

10. A pharmaceutical composition according to claim 7 or 8 for use in inhibiting cancer induced by HPV16.

## Patentansprüche

1. Fusionsprotein aufweisend:
a) ein Polypeptid, aufweisend ein *Pseudomonas* Exotoxin A (PE)-Fragment, wobei das PE-Fragment die Domänen I und II umfasst, aber nicht die Domänen III des Exotoxins;
b) ein menschliches Papillom-Virus Typ 16 (HPV16) E7 Protein; und
c) einen Carboxyl-Endabschnitt, der eine Aminosäuresequenz aufweist, ausgewählt aus der Gruppe bestehend aus KDEL, KDELRDELKDEL und SEQ ID NO: 2, wobei die Großbuchstaben, die in den Begriffen 'KDEL' und 'KDELRDELKDEL' verwendet werden, Einzelbuchstaben-Aminosäurecodes bezeichnen.

2. Fusionsprotein nach Anspruch 1, wobei der Carboxyl-Endabschnitt die Aminosäuresequenz von KDELRDELKDEL aufweist.

3. Fusionsprotein gemäß Anspruch 1 oder 2, wobei das HPV 16 E7 Protein die Aminosäuresequenz der SEQ ID NO: 3 aufweist.

4. Fusionsprotein nach Anspruch 1, wobei der Carboxyl-Endabschnitt die Aminosäuresequenz KDEL aufweist.

5. Fusionsprotein gemäß Anspruch 1, wobei der Carboxyl-Endabschnitt die Aminosäuresequenz von SEQ ID NO: 2 aufweist.

6. Pharmazeutische Zusammensetzung, die eine wirksame Menge eines Fusionsproteins gemäß irgendeinem der Ansprüche 1 - 5 aufweist, um Krebs, der durch HPV 16 induziert wurde, zu unterdrücken.

7. Pharmazeutische Zusammensetzung gemäß Anspruch 6, die ferner ein Hilfsmittel aufweist.

8. Pharmazeutische Zusammensetzung gemäß Anspruch 7, wobei das Hilfsmittel ein Öl-Hilfsstoff ist.

9. Fusionsprotein gemäß irgendeinem der Ansprüche 1 - 5 zur Verwendung bei der Inhibierung von Krebs, induziert durch HPV 16.

10. Pharmazeutische Zusammensetzung gemäß Anspruch 7 oder 8 zur Verwendung bei der Inhibierung von Krebs, der durch HPV 16 induziert wurde.

## Revendications

1. Protéine de fusion comprenant :
a) un polypeptide comprenant un fragment de l'exotoxine A de *Pseudomonas* (PE), le fragment PE comprenant les domaines I et II mais pas le domaine III de l'exotoxine ;
b) une protéine E7 du papillomavirus humain de type 16 (HPV16) ; et
c) une partie carboxy-terminale comprenant une séquence d'acides aminés sélectionnée dans le groupe consistant en KDEL, KDELRDELKDEL et SEQ ID NO:2, où les lettres majuscules utilisées dans les termes 'KDEL' et 'KDELRDELKDEL' représentent des codes d'acides aminés à une lettre.

2. Protéine de fusion selon la revendication 1, dans laquelle la partie carboxy-terminale comprend la séquence d'acides aminés de KDELRDELKDEL.

3. Protéine de fusion selon la revendication 1 ou 2, dans laquelle la protéine E7 du HPV16 comprend la séquence d'acides aminés de SEQ ID NO:3.

4. Protéine de fusion selon la revendication 1, dans laquelle ladite partie carboxy-terminale comprend la séquence d'acides aminés de KDEL.

5. Protéine de fusion selon la revendication 1, dans laquelle la partie carboxy-terminale comprend la séquence d'acides aminés de SEQ ID NO: 2.

6. Composition pharmaceutique comprenant une quantité efficace d'une protéine de fusion selon l'une quelconque des revendications 1 à 5, pour supprimer un cancer induit par le HPV16.

7. Composition pharmaceutique selon la revendication 6, comprenant en outre un adjuvant.

8. Composition pharmaceutique selon la revendication 7, dans laquelle l'adjuvant est un adjuvant huileux.

9. Protéine de fusion selon l'une quelconque des revendications 1 à 5, destinée à être utilisée pour inhiber un cancer induit par le HPV16.

10. Composition pharmaceutique selon la revendication 7 ou 8, destinée à être utilisée pour inhiber un cancer induit par le HPV16.
